# EUROPEAN PATENT SPECIFICATION

(11) **EP 1 444 247 B1**
(45) Date of publication and mention of the grant of the patent: **13.06.2007**
(21) Application number: 02784184.0
(22) Date of filing: 18.10.2002
(51) Int. Cl.: C07J 7/00, A61K 31/56, A61P 5/30

(54) **TIBOLONE FORMULATIONS**
TIBOLONFORMULIERUNGEN
FORMULATIONS DE TIBOLONE

(30) Priority: 18.10.2001 GB 0125061; 02.11.2001 US 350121 P
(43) Date of publication of application: 11.08.2004
(73) Proprietor: NORTON HEALTHCARE LIMITED, London E16 2QJ (GB)
(72) Inventor: BRENNAN, Tom, Clonmel, Co. Tipperary (IE); WOOLFE, Austin John, Essex CM16 DL (GB)
(74) Representative: Schnappauf, Georg
(86) International application number: PCT/US2002/033526
(87) International publication number: WO 2003/032924

(56) References cited:
- US-A- 4 701 450
- US-A- 5 037 817
- US-A- 5 512 556

## Description

### FIELD OF THE INVENTION

The invention relates to stabilized pharmaceutical compositions and dosage forms of tibolone (7α,17α)-17-hydroxy-7-methyl-19-nor-17-pregn-5(10)-en-20-yn-3-one.

### BACKGROUND OF THE INVENTION

Tibolone (C₂₁H₂₈O₂) is a synthetic steroid of the oestrane series (a Δ⁵⁽¹⁰⁾-oestrene derivative) known to have combined oestrogenic, progestogenic and androgenic characteristics. Tibolone is structurally related to the progestogens norethindrone and norethynodrel. For a general review of the pharmacology of tibolone see van der Vies, Maturitas Suppl. 1:15-24 (1987). Tibolone is used *inter alia,* in pharmacological preparations having gonadomimetic, ovulation-inhibiting or immuno-modulatory action (see Tax et al., Progress in Basic Clinical Pharmacology 6:143-159 (1991) and Tax et al., Maturitas Suppl. 1:3-13 (1987) for a general review of the clinical pharmacology of tibolone).
US 5,512,556 describes the use of particular pregnane derivatives, in particular (7α,17α)-17-hydroxy-7-methyl-19-nor-17-pregn-5(10)-en-20-yn-3-one, for the manufacture of a medicament for the prevention or treatment of tumors.

Unfortunately, tibolone dosage forms (e.g., tablets or capsules) are both difficult to prepare and are plagued by instability problems. One of the tibolone polymorphs generally found in tibolone preparations, particularly in preparations which are not enriched for a particular polymorph, has been shown to be difficult to dissolve. In addition, the resulting formulations obtained generally suffer from limited storage stability especially under dry conditions. The inherent stability is due to the presence of an impurity (*i.e*., (7α,17α)-17-hydroxy-7-methyl-19-nor-17-pregn-4-en-20-yn-3-one), which increases during the preparation of pharmaceutical dosage units. Unfortunately, the amount of the destabilizing impurity also increases during storage by conversion of (7α,17α)-17-hydroxy-7-methyl-19-nor-17-pregn-5(10)-en-20-yn-3-one into (7α,17α)-17-hydroxy-7-methyl-19-nor-17-pregn-4-en-20-yn-3-one by acid catalyzed isomerization.

Various attempts have been reported to try to overcome these problems. Hence, for example EP 159739 discloses a number of tablet formulations of tibolone containing conventional tablet excipients. EP 159739 however, does not address the stability problems associated with tibolone formulations.

EP 389035 describes the production of two pure polymorphic forms (forms I and II) of tibolone. This patent further postulates that polymorph I is appreciably more stable than the polymorph II. Allegedly more stable preparations comprising a crystalline pure or virtually pure form which is completely or, virtually completely free from the other crystalline form are disclosed and are presently marketed under the mark LIVIAL^{™} in the United Kingdom.

WO 98/47517 describes the use of a high percentage (above 10%) of starch in a tibolone formulation and claims that better stability is obtained, particularly under relatively dry storage conditions or with lower doses of tibolone.

Although the approaches found in the art may to some extent improve some of the problems still associated with tibolone formulation (*i.e.*, stability and solubility), there remains a need to identify compositions and methods better suited to arrive to stable as well as soluble tibolone preparations.

### SUMMARY OF THE INVENTION

The invention meets the present needs by providing a composition comprising tibolone in admixture with one or more excipients, and a pH adjusting agent which is a salt of a polybasic acid, wherein the polybasic acid is citric, tartaric, fumaric, maleic or succinic.

An additional aspect of the invention relates to the use of a pH adjusting agent which is a salt of a polybasic acid in a composition comprising tibolone in admixture with one or more excipients for the stability of tibolone in said composition.
Preferred embodiments of the composition and use of the present invention are defined in claims 2 to 6 and 8 to 17, respectively.

### BRIEF DESCRIPTION OF THE DRAWINGS

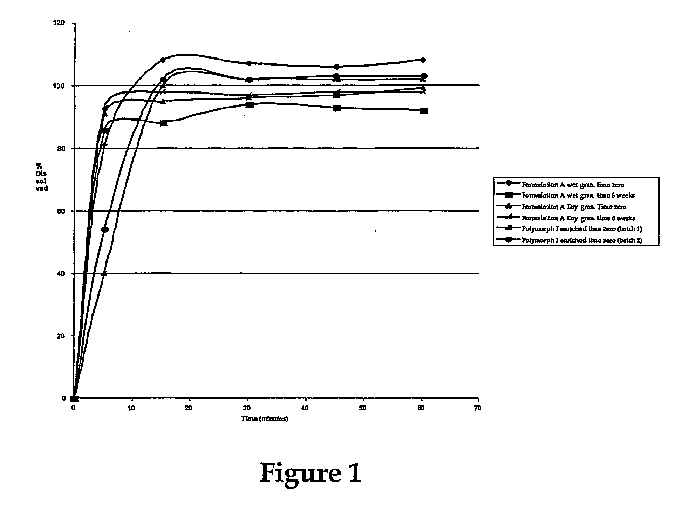

Figure 1 is a representation of a graph showing the dissolution profiles of (a) representative formulations RDT0328 prepared by wet granulation, and RDT0329 prepared by dry granulation, according to the invention (see specifics for Formulation A in example 2 hereinafter) at time 0 and after 6 weeks of storage at 40°C and 75 % relative humidity, and of (b) IP0022 and IP0047, both are polymorph I-enriched formulations (see EP 389035).

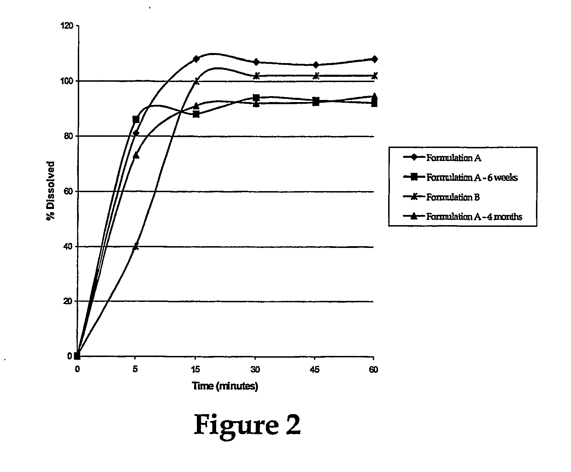

Figure 2 is a representation of a graph showing the stability profiles of (a) representative Formulation A according to the invention (see Example 2 hereinafter) at time 0, after 6 weeks, and after 4 months of storage at 40°C and 75% relative humidity, and of (b) Formulation B which is polymorph I-enriched (see EP 389035).

### DETAILED DESCRIPTION OF THE INVENTION

Surprisingly, it has now been discovered that the inclusion of a pH-adjusting agent which is a salt of a polybasic acid increases the stability of tibolone formulations.

Moreover, it has been found that even formulations containing more than threshold percentages of polymorph II -which has been reported in the literature to be associated with instability and solubility problems- may be stabilized by the inclusion of a pH adjusting agent in the formulation. The aim of the present invention is therefore to obtain compositions which are stable, soluble, and do not necessarily require pure or quasi-pure crystalline preparations.

The patents, published applications, and scientific literature referred to herein establish the knowledge of those with skill in the art and are hereby incorporated by reference in their entirety to the same extent as if each was specifically and individually indicated to be incorporated by reference. Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter.

Any suitable materials and/or methods known to those of skill can be utilized in carrying out the present invention. However, preferred materials and methods are described. Materials, reagents and the like to which reference is made in the following description and examples are obtainable from commercial sources, unless otherwise noted.

Technical and scientific terms used herein have the meaning commonly understood by one of skill in the art to which the present invention pertains, unless otherwise defined. Reference is made herein to various methodologies and materials known to those of skill in the art. Standard works setting forth the general principles of pharmaceutical dosage form preparation techniques including granulation, mixing, coating techniques include for example, Lachman et al. Eds., The Theory and Practice of Industrial Pharmacy, 3rd Ed., (1986), and Lieberman et al., Eds. Pharmaceutical Dosage Forms, Marcel Dekker Inc., New York and Basel (1989).

As used in this specification, the singular forms "a," "an" and "the" specifically also encompass the plural forms of the terms to which they refer, unless the content clearly dictates otherwise. Similarly, in the specification and the appended claims, the singular forms include plural referents unless the context clearly dictates otherwise.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least." When used in the context of a process, the term "comprising" means that the process includes at least the recited steps, but may include additional steps. When used in the context of a compound or composition, the term "comprising" means that the compound or composition includes at least the recited features or components, but may also include additional features or components.

The invention provides pharmaceutical compositions containing tibolone in admixture with one or more excipients, and a pH adjusting agent. The term "pH adjusting agent" is used to denote an agent which when mixed with one or more compounds in a formulation adjusts the acidity of the formulation and may additional have antioxidative properties. In some embodiments, the pH adjusting agent may have synergistic antioxidative properties with other compounds in the formulation to which the pH adjusting agent is added. The pH adjusting agent used according to the invention is a salt of a polybasic acid, particularly a weak polybasic acid, such as a carboxylic acid. Suitable salts of carboxylic acids include salts of citric, fumaric, tartaric, maleic, or succinic acid. Particular examples of pH adjusting agents are the acid salts of citric acid, for example monosodium dihydrogen citrate, disodium hydrogen citrate and especially sodium citrate. The polybasic acid may also be an inorganic polybasic acid. Salts of inorganic polybasic acids that may be mentioned include phosphate, hydrogen phosphate, carbonate and hydrogen carbonate, in particular potassium and especially sodium hydrogen carbonate (also known as sodium bicarbonate). Borates, for example sodium borate, may also be mentioned.

The cation of the salt of the polybasic acid may be inorganic or organic. Suitable inorganic cations include the alkali metals, e.g., sodium and potassium, and the alkali earths, in particular magnesium and calcium. Organic cations include quaternary ammonium salts.

One of skills in the art will appreciate that the compositions of the invention may be solid as well as liquid depending on the specific exigencies and circumstances.

In certain embodiments the compositions of the invention are liquid compositions comprising a 1%w/v aqueous solution of the pH adjusting agent having a pH of from about 4 to about 10, more preferably from about 6 to about 9 and most preferably from about pH 7 to about pH 9.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Examples of pH of a 1%w/v aqueous solutions of suitable pH adjusting agents are as follows:

**Table I**

| | | |
|---|---|---|
| 1%w/v aqueous solution | Sodium Bicarbonate | pH 8.2 |
| 1%w/v aqueous solution | Sodium Citrate | pH 8.75 |

Insofar as both solid as well as liquid compositions are contemplated, it is understood that the pH adjusting agent used according to the invention may be in a solid form such as a crystalline or a dry/fine powder form.

The dosage unit forms exemplified hereinafter include 2.5 mg of tibolone in a tablet form or 100 mg of a pharmaceutically acceptable powder in capsules (*i.e*., 2.5%). However, there is a long-felt need to provide lower dosage forms to fine-tune therapeutic regimens to individual patients' needs. Unfortunately, simply lowering the tibolone content results in a dramatic and prohibitive decrease in stability and concurrent shelf life. Inclusion of the pH adjusting agent used according to the invention is therefore also useful to stabilize formulations having a low (i.e., less than 2.5 mg) tibolone content.

In certain embodiments of the invention, the ratio of pH adjusting agent to tibolone is from about 10 parts agent to about 1 part tibolone to about 0.01 parts agent to about 1 part tibolone.

For oral administration, the compositions of the invention may be presented as discrete units such as capsules, caplets, gelcaps, cachets, pills, or tablets each containing a predetermined amount of the active ingredient as a powder or granules; as a solution or a suspension in an aqueous liquid or a non-aqueous liquid; or as an oil-in-water liquid emulsion or a water-in-oil emulsion and as a bolus, *etc*.

Alternately, administration of a composition of all of the aspects of the present invention may be effected by liquid solutions, suspensions or elixirs, powders, lozenges, micronized particles and osmotic delivery systems. Pharmaceutical dosage forms especially contemplated in the present invention include solid dosage forms such as tablets or capsules, as well as other dry (non-solid) or liquid forms. One of skill in the art will appreciate that the compositions of the invention are easily adapted without undue experimentation for administration by other routes.

A pharmaceutical oral dosage form, such as for example a tablet, may be made by a variety of methods known in the art (see e.g., Lachman *et al.* (supra) and Lieberman *et al. (supra)),* e.g. by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface active or dispersing agent. Molded tablets may be made by molding, in a suitable machine, a mixture of the powdered compound moistened with an inert liquid diluent. The tablets may be optionally coated or scored and may be formulated to provide a slow or controlled release of the active ingredient therein.

The methods set forth in the examples provided hereinafter are merely representative examples to illustrate some of the possible ways available and are not meant to limit the scope of the invention.

The compositions according to the invention may be prepared by the simple addition of the active agent (*i.e*., tibolone) to the powder, tablet, capsule or granule mix with all the other components described herein.

In a further embodiment, tibolone and the pH adjusting agent are mixed together and then granulated with a solution of binder in water or organic solvent e.g., an alcohol. Any auxiliary components e.g., starch may be mixed subsequently as well. The binder may in principle be any suitable pharmaceutical binder such as any cellulose derivative e.g., hydroxypropyl methyl cellulose or polymers such as polyvinyl pyrrolidone. In certain embodiments, the compositions of the invention may further include components useful to achieve the release of tibolone over time or to delay the release of tibolone (e.g., extended release, continued release or delayed release) such as for example hydroxypropyl methylcellulose, hydroxypropyl cellulose, ethyl cellulose, hydroxyethyl cellulose, castor oils, vegetable oils, xanthan gum, and waxes.

In some embodiments, the compositions of the invention further comprise antioxidant compounds such as for example vitamin E, vitamin C, carotene, ascorbyl palmitate, ascorbyl stearate, propylgallate, lactic acid, and erythrobic acid.

In particular, a starch paste or other starch derivative is particularly preferred. In addition, the binder compound could be added to the dry mix and granulated with pure water or solvent.

It will be understood that in all embodiments of this invention conventional pharmaceutical excipients can be added to the compositions.

The compositions according to the invention are optionally formulated with any of the well known pharmaceutically acceptable carriers, including diluents and excipients (see Remington's Pharmaceutical Sciences 18th Ed., Gennaro, Mack Publishing Co., Easton, PA 1990, Remington: The Science and Practice of Pharmacy, Lippincott, Williams & Wilkins, 1995, and The Handbook of Pharmaceutical Excipients, 3rd Ed., American Pharmaceutical Association and Pharmaceutical Press, 2000). This includes bulking agents such as sugars, e.g., lactose; cellulose derivatives e.g., microcrystalline cellulose; calcium salts e.g., calcium phosphate or calcium sulphate; disintegrants e.g., sodium starch glycolate, crosscarmellose; lubricants e.g., magnesium stearate, sodium stearyl fumarate and surfactants, and wetting agents, e.g., sodium lauryl sulphate, conventional poloxamers, polyethylene glycols, sodium tetradecylsulphate, and sorbitan esters.

Other pharmaceutical excipients such as colors, flavors, etc. may also be added. While the type of pharmaceutically acceptable carrier/vehicle employed in generating the compositions of the invention will vary depending upon the mode of administration of the composition to a mammal, generally pharmaceutically acceptable carriers are physiologically inert and non-toxic. Formulations of compositions according to the invention may contaih more than one active ingredient as well any other pharmacologically active ingredient useful for the treatment of the symptom/ condition being treated.

### EXAMPLES

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein.

### Example 1

### Preparation of Tibolone/Sodium Bicarbonate Formulations

This example is provided to illustrate the preparation of a representative solid form tibolone formulation as described in more details heretofore, 2.5g of tibolone (obtained from Chemo Iberia, Italy) was admixed and triturated with 2.5g of sodium bicarbonate (Industriale Chimica s.r.1, Spain). This preparation was subsequently blended with 84g of lactose (Merck KGaA, Germany), 10g of starch (Colorcon, UK), and 1 g of magnesium stearate (Merck KGaA, Germany). The blend was then compressed into tablets of 100mg each, containing 2.5mg of tibolone. Samples were analyzed (λ= 200-240nm) to identify conjugated impurities (here identified as RC01 and RC04).

**Table II**

| | RC04 | RC01 | TOTAL IMPURITY |
|---|---|---|---|
| Formulation without Bicarbonate Sodium | 1.32% | 2.98% | 7.47% |
| 100g batch with Sodium Bicarbonate | 1.69% | 1.48% | 5.61% |

### Example 2

### Preparation of Tibolone/Sodium Citrate Formulations

This example is provided to illustrate the preparation of representative tibolone blends as described in more details heretofore. 2.5g of tibolone (obtained from Industriale Chimica SRL, Italy) was admixed and triturated with 2.5g of sodium citrate (Merck KgaA, Germany) and 5g of starch. This preparation was subsequently granulated with 5% solution of hydroxypropyl methyl cellulose in water, and then dried. Granules were then milled and dry blended with 85g of lactose, 2g of sodium starch glycolate, and 1g of magnesium stearate. The blend was then compressed into tablets of 100mg each, containing 2.5mg of tibolone. Samples were analyzed (λ= 200-240nm) to identify conjugated impurities (here identified as RC01 and RC04).

**Table III**

| | RC04 | RC01 | TOTAL IMPURITY |
|---|---|---|---|
| Formulation without Sodium Citrate | 1.32% | 2.98% | 7.47% |
| 100g batch with Sodium Citrate | 1.78% | 1.22% | 5.29% |
| 400g batch with Sodium Citrate | 1.58% | 1.62% | 5.65% |

In another experiment a tibolone/sodium citrate formulation was prepared essentially as described in the above paragraph and further comprising standard pharmaceutical excipients as described in more details above. The final 2.5 mg tibolone unit dose (Formulation A) form included:

**Table IV**

| **Formulation A Composition** | | | |
|---|---|---|---|
| Tibolone | | | 2.5mg |
| Lactose | | | 86.1mg |
| | Lactose monohydrate (200 mesh) | 52.775 | |
| | Spray dried lactose | 33.33 | |
| | (dried compression grade) | | |
| Pregelatinized starch | | | 8.0mg |
| Ascorbyl palmitate | | | 0.2mg |
| Sodium Citrate | | | 0.69mg |
| Sodium Lauryl Sulphate | | | 0.005mg |
| Croscarmellose Sodium | | | 2.0mg |
| Mg Stearate | | | 0.5mg |

### Example 3

### Solubility of Tibolone Preparations

It has been observed that tibolone preparations which have not been enriched for a particular polymorph (i.e., polymorph I) are plagued by an inherent lower solubility hindering their value for pharmaceutical purposes. To ascertain that the compositions of the invention are soluble, a series of dissolution tests were performed including the one included hereinafter for illustrative purposes. In this experiments, the % dissolution for Formulation A (see Example 2, above) and Formulation B which is polymorph I-enriched (see EP 389035) were compared (for both wet and dry granulation methodologies).

As shown in Figure 1, the dissolution profiles of (a) representative formulations RDT0328 prepared by wet granulation, and RDT0329 prepared by dry granulation, according to the invention (see specifics for Formulation A in example 2, above) at time 0 and after 6 weeks of storage at 40°C and 75% relative humidity, were comparable to the dissolution profiles observed for (b) Formulation B samples IP0022 and IP0047 (both are polymorph I-enriched formulations (see EP 389035)).

### Example 4

### Analytical Evaluation of Tibolone Preparations-Stability

This example is provided to evidence the stability of the compositions according to the invention. For this purpose, the tibolone preparation Formulation A of Example 3 above was analyzed and further characterized. It was established that the tibolone of the preparation of Example 3 had a polymorphic ratio of 85% form I, and 15% of form II. It was found that there was no detectable transition from form II to form I upon prolonged storage. To test and compare the stability of the formulations according to the invention, Formulation A according to the invention (see Example 3, *supra*) was tested at time 0, after 6 weeks, and after 4 months of storage at 40°C and 75% relative humidity, and compound to Formulation B which is polymorph I-enriched (see EP 389035).

The specific dissolution parameters for this set of experiments were as follows:

| | |
|---|---|
| Apparatus: | Paddles |
| Rotation Speed: | 75rpm |
| Dissolution Medium: | 0.25% w/w sodium dodecyl (Lauryl) |
| | sulphate |
| Medium Volume: | 900ml |
| Medium Temperature: | 37°C ± 0.5°C |
| Detection: | UV @ 210nm |
| Sampling Times: | 15mins |

**Table V**

| | FORMULATION | | FORMULATION A | | |
|---|---|---|---|---|---|
| | B | TIME ZERO | 3 WEEKS | 6 WEEKS | 4 MONTHS |
| ASSAY | 96.9% | 112.4% | 99.3% | 96.7% | 95.3% |
| | (99.7mg) | (110,0mg) | (97.6mg) | (95.3mg) | (91.7mg) |
| DISSOLUTION | 100% | 108% | N/ T | 88% | 91% |
| AT 15 MINS | (100mg) | (106mg) | | (92.5rng) | (95.0mg) |
| TOTAL RELATED SUBSTANCE | 5.21% | 1.03% | 1.57% | 1.34% | 3.2% |

**[047]** As shown in Figure 2, the stability of Formulation A preparations according to Example 3 were comparable to that observed for polymorph I-enriched preparations reported to be stable (see EP 389035) even after 4 months under accelerated storage conditions.

Similarly, sample formulations matching those described in Examples 1 and 2 but omitting the pH adjusting agents (*i.e*., sodium citrate and sodium bicarbonate) were included as well as samples as negative controls. These tests also showed that the presence of the pH adjusting agents stabilized the formulations (data not shown).

### Example 5

### Pharmacokinetic Studies of Representative Tibolone Compositions

To illustrate the properties of the compositions according to the invention, in a pilot study, several subjects received two single oral administration of 2.5mg tibolone as the test and reference formulations according to a cross-over design. Each administration was separated by a two weeks wash out period. Standard bioavailability studies demonstrate that the active ingredient (i.e., tibolone) in the compositions disclosed herein reaches its maximal concentration (tₘₐₓ) within published ranges, that it is also biologically converted to its isomeric form, and that the AUC values are within accepted ranges for bioequivalence (data not shown). Pharmacokinetic parameters for tibolone and Δ4-tibolone are calculated according to standard methods described in the literature.

### Equivalents

Reference is made hereinafter in detail to specific embodiments of the invention. While the invention is described in conjunction with these specific embodiments, it will be understood that it is not intended to limit the invention to such specific embodiments. On the contrary, it is intended to cover alternatives, modifications, and equivalents as may be included within the spirit and scope of the invention as defined by the appended claims. In the instant description, numerous specific details are set forth in order to provide a thorough understanding of the present invention. The present invention may be practiced without some or all of these specific details. In other instances, well known process operations have not been described in detail, in order not to unnecessarily obscure the present invention.

## Claims

1. A composition comprising tibolone in admixture with one or more excipients, and a pH adjusting agent which is a salt of a polybasic acid, wherein the polybasic acid is citric, tartaric, fumaric, maleic or succinic.

2. A composition according to claim 1, wherein the cation of the salt is an inorganic cation.

3. A composition according to claim 2, wherein the inorganic cation is sodium, potassium, magnesium or calcium.

4. A composition according to claim 1, wherein the cation of the salt is an organic cation.

5. A composition according to claim 1, wherein the composition is a solid dosage form.

6. A composition according to claim 1, further comprising a binder selected from the group consisting of starch, pre-gelatinized starch, hydroxypropyl cellulose, hydroxypropyl methyl cellulose and polyvinyl pyrrolidone.

7. Use of a pH adjusting agent which is a salt of a polybasic acid in a composition comprising tibolone in admixture with one or more excipients for the stability of tibolone in said composition.

8. Use according to claim 7, wherein the polybasic acid is a weak acid.

9. Use according to claim 8, wherein the polybasic acid is a carboxylic acid.

10. Use according to claim 7 or 8, wherein the polybasic acid is citric, tartaric, fumaric, maleic or succinic.

11. Use according to claim 7, wherein the polybasic acid is an inorganic polybasic acid.

12. Use according to claim 11, wherein the salt of the polybasic acid is a phosphate salt, a hydrogen phosphate salt, a sulphate salt, a hydrogen sulphate salt, a carbonate salt or a hydrogen carbonate salt.

13. Use according to claim 7, wherein the cation of the salt is an inorganic cation.

14. Use according to claim 13, wherein the inorganic cation is sodium, potassium, magnesium or calcium.

15. Use according to claim 7, wherein the cation of the salt is an organic cation.

16. Use according to claim 7, wherein the composition is a solid dosage form.

17. Use according to claim 7, wherein the composition further comprises a binder selected from the group consisting of starch, pre-gelatinized starch, hydroxypropyl cellulose, hydroxypropyl methyl cellulose and polyvinyl pyrrolidone.

## Patentansprüche

1. Zusammensetzung, die Tibolon zusammen mit einem oder mehreren Exzipienzien und ein pH-Einstellungsmittel, das ein Salz einer polybasischen Säure ist, umfasst, wobei die polybasische Säure Zitronen-, Wein-, Fumar-, Malein- oder Bernsteinsäure ist.

2. Zusammensetzung gemäß Anspruch 1, wobei das Kation des Salzes ein anorganisches Kation ist.

3. Zusammensetzung gemäß Anspruch 2, wobei das anorganische Kation Natrium, Kalium, Magnesium oder Calcium ist.

4. Zusammensetzung gemäß Anspruch 1, wobei das Kation des Salzes ein organisches Kation ist.

5. Zusammensetzung gemäß Anspruch 1, wobei die Zusammensetzung in einer Form einer festen Dosierung vorliegt.

6. Zusammensetzung gemäß Anspruch 1, die ferner ein Bindemittel umfasst, ausgewählt aus der Gruppe, bestehend aus Stärke, vorgelatinisierter Stärke, Hydroxypropylcellulose, Hydroxypropylmethylcellulose und Polyvinylpyrrolidon.

7. Verwendung eines pH-Einstellungsmittels, das ein Salz einer polybasischen Säure ist, in einer Zusammensetzung, die Tibolon zusammen mit einem oder mehreren Exzipienzien umfasst, für die Stabilität von Tibolon in der Zusammensetzung.

8. Verwendung gemäß Anspruch 7, wobei die polybasische Säure eine schwache Säure ist.

9. Verwendung gemäß Anspruch 8, wobei die polybasische Säure eine Carbonsäure ist.

10. Verwendung gemäß Anspruch 7 oder 8, wobei die polybasische Säure Zitronen-, Wein-, Fumar-, Malein- oder Bernsteinsäure ist.

11. Verwendung gemäß Anspruch 7, wobei die polybasische Säure eine anorganische polybasische Säure ist.

12. Verwendung gemäß Anspruch 11, wobei das Salz der polybasischen Säure ein Phosphatsalz, ein Hydrogenphosphatsalz, ein Sulfatsalz, ein Hydrogensulfatsalz, ein Carbonatsalz oder ein Hydrogencarbonatsalz ist.

13. Verwendung gemäß Anspruch 7, wobei das Kation des Salzes ein anorganisches Kation ist.

14. Verwendung gemäß Anspruch 13, wobei das anorganische Kation Natrium, Kalium, Magnesium oder Calcium ist.

15. Verwendung gemäß Anspruch 7, wobei das Kation des Salzes ein organisches Kation ist.

16. Verwendung gemäß Anspruch 7, wobei die Zusammensetzung in einer Form einer festen Dosierung vorliegt.

17. Verwendung gemäß Anspruch 7, wobei die Zusammensetzung ferner ein Bindemittel umfasst, ausgewählt aus der Gruppe, bestehend aus Stärke, vorgelatinisierter Stärke, Hydroxypropylcellulose, Hydroxypropylmethylcellulose und Polyvinylpyrrolidon.

## Revendications

1. Composition comprenant de la tibolone en mélange avec un ou plusieurs excipients, et un agent d'ajustement du pH qui est un sel d'un polyacide, tandis que le polyacide est l'acide citrique, tartrique, fumarique, maléique ou succinique.

2. Composition selon la revendication 1, dans laquelle le cation du sel est un cation inorganique.

3. Composition selon la revendication 2, dans laquelle le cation inorganique est sodium, potassium, magnésium ou calcium.

4. Composition selon la revendication 1, dans laquelle le cation du sel est un cation organique.

5. Composition selon la revendication 1, dans laquelle la composition est une forme galénique solide.

6. Composition selon la revendication 1, comprenant en outre un liant choisi dans le groupe consistant en amidon, amidon pré-gélatinisé, hydroxypropyl cellulose, hydroxypropyl méthyl cellulose et poly(pyrrolidone de vinyle).

7. Utilisation d'un agent d'ajustement du pH qui est un sel d'un polyacide dans une composition comprenant de la tibolone en mélange avec un ou plusieurs excipients pour la stabilité de la tibolone dans ladite composition.

8. Utilisation selon la revendication 7, dans laquelle le polyacide est un acide faible.

9. Utilisation selon la revendication 8, dans laquelle le polyacide est un acide carboxylique.

10. Utilisation selon la revendication 7 ou 8, dans laquelle le polyacide est l'acide citrique, tartrique, fumarique, maléique ou succinique.

11. Utilisation selon la revendication 7, dans laquelle le polyacide est un polyacide inorganique.

12. Utilisation selon la revendication 11, dans laquelle le sel du polyacide est un sel phosphate, un sel hydrogéno phosphate, un sel sulfate, un sel hydrogéno sulfate, un sel carbonate ou un sel hydrogéno carbonate.

13. Utilisation selon la revendication 7, dans laquelle le cation du sel est un cation inorganique.

14. Utilisation selon la revendication 13, dans laquelle le cation inorganique est sodium, potassium, magnésium ou calcium.

15. Utilisation selon la revendication 7, dans laquelle le cation du sel est un cation organique.

16. Utilisation selon la revendication 7, dans laquelle la composition est une forme galénique solide.

17. Utilisation selon la revendication 7, dans laquelle la composition comprend en outre un liant choisi dans le groupe consistant en amidon, amidon pré-gélatinisé, hydroxypropyl cellulose, hydroxypropyl méthyl cellulose et poly(pyrrolidone de vinyle).
